# EUROPEAN PATENT APPLICATION

(11) **EP 1 428 542 A2**
(43) Date of publication of application: **16.06.2004**
(21) Application number: 03028345.1
(22) Date of filing: 10.12.2003
(51) Int. Cl.: A61M 5/00, A61M 5/32

(54) **Sterile container**

(30) Priority: 13.12.2002 IT fi20020246
(71) Applicant: MOLTENI L. & C. dei Fratelli Alitti Società di Esercizio S.P.A., 50018 Scandicci (Firenze) (IT)
(72) Inventor: Angeli, Roberto, 50018 Scandicci (IT); Bonifacio, Marco, 53034 Colle di Val d'Elsa (IT)
(74) Representative: Gervasi, Gemma, Dr.

(57) **Abstract**

A container providing prolonged sterility of its content consists of a cylindrical body (10) with an open end an annular recess (15) near the open end and a closure cap (11) with an annular rib (14). Recess (15) and rib (14) are arranged to mate with each other when the cap is plugged onto the container providing a hermetic seal.
Also disclosed is an assembly for cannulae with a short cannula (21) telescopically arranged inside a long cannula (20). The short cannula disposes a spike for transferring a medical formulation from a package to either the long or the short cannula. A hub portion of the short cannula has an annular projection (30) interacting with an annular recess (25) on the inner surface of the proximal portion of the long cannula providing a hermetic seal.
A kit is proposed consisting of the container and the cannula assembly.

## Description

### Field of the invention

The present invention relates to the field of sterile containers.

### Prior Art

As is known in the case of instruments to be utilized for the application or administration of drugs, it is of fundamental importance to guarantee their sterility until the time of use.

This is particularly important when the instruments considered are of the disposable type and are used directly by the patient without the aid of skilled personnel.

Considering that these instruments are often kept for long periods of time their containers must allow a perfect seal in order to guarantee the sterility of its content until the time of use.

The importance of having a container that although simple and inexpensive also guarantees the required security and sterility is therefore evident.

### Summary of the invention

The invention therefore relates to a sterile container comprising:
a hollow body provided with an aperture
a sealed cap able to become engaged in the aforesaid aperture and to guarantee total sterility of the contents of the aforesaid hollow body.

### Description of the figures

Figure 1 shows a container according to the invention (1a) with its respective section (1 b);
Figure 2 shows a particular embodiment of a container according to the invention (2a) with its respective section (2b);
Figure 3 shows a cannula of the disposable type, constituted by two parts (shown separated in the figure).
Figure 4 shows a section of the two parts of the cannula shown in Figure 3 (also shown separated here).

### Detailed description of the invention

The present invention makes it possible to overcome the aforesaid problems by providing a container that thanks to the particular interaction between body and cap is able to guarantee total sterility of the content even for long periods of time (years).

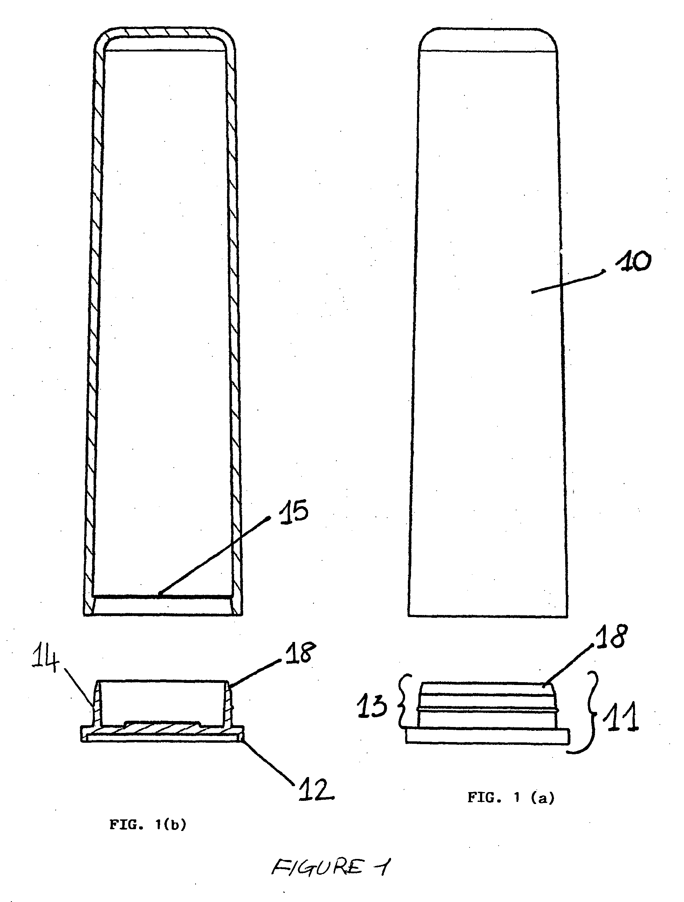

As can be seen in Figure 1, a container according to the invention is constituted by an essentially cylindrical body 10, hollow and open at only one end, and by a cap 11 capable of engaging with perfect seal in the aperture of the body 10.

The cap 11 has a head 12 and a cylindrical extension 13, with a smaller diameter than the head 12, on the outer surface of which is a raised circular part 14.

The difference between the radius of the head 12 and the radius of the cylindrical extension 13 is equal to the thickness of the body part 10 that therefore acts as a stop for the cap.

The final part 18 of the cylindrical extension 13 is slightly tapered to facilitate fitting of the cap.

On the inner surface of the body 10 is a raised part 15, disposed at a distance from the edge slightly below the distance between the lower surface of the head 12 of the cap 11 and the raised circular part 14.

The raised parts 14 and 15 must be even slightly elastically deformable so that when the cap is inserted into the aperture of the hollow body 10 the pressure exerted by the closing action allows the raised circular part 14 to pass over the raised part 15 thereby engaging with it.

It is immediately evident that this simple expedient guarantees perfect seal, as the closing action exerted by the lower surface of the head 12 of the cap 11 which adheres to the upper surface of the walls of the body 10 (as is the case in known containers) is added to the closing action exerted by the raised parts 14 and 15 overlapping and engaging with each other.

In fact, experimental tests performed show that sterile products contained in a container according to the invention remained sterile even for several years.

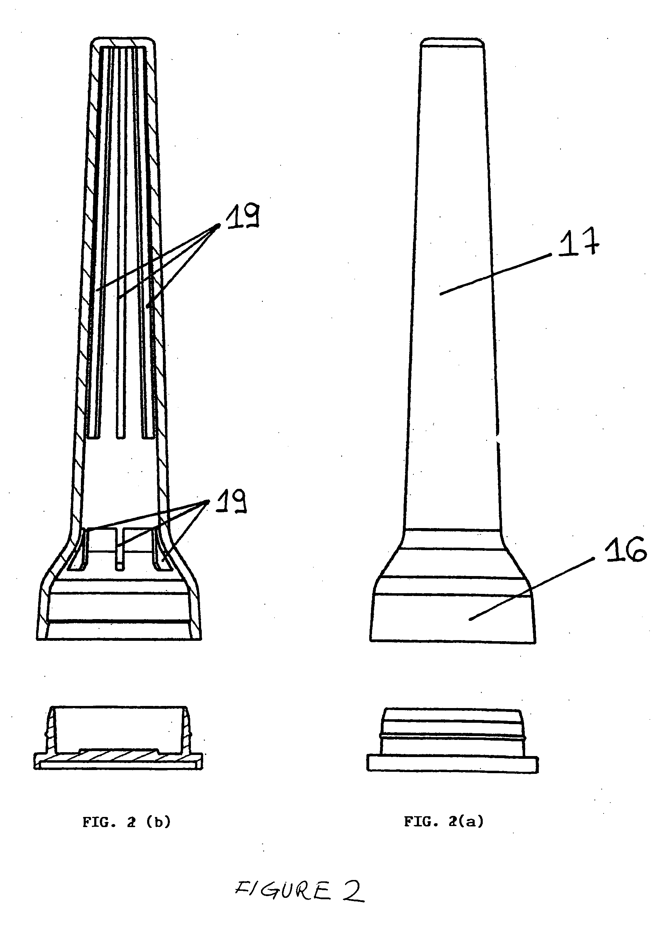

According to a particular embodiment of the invention, as shown in Figure 2, the hollow cylindrical body 10 is shaped so that the open end 16 has the dimensions of the cylindrical extension 13 of the cap 11 subsequently tapering to adopt the shape of a cannula 17.

The container according to this embodiment is particularly suitable to keep cannulae sterilised, for example disposable cannulae, for long periods of time.

In this case, moreover, the inner surface of the parts 16 and of the intermediate and final part of the part 17 may have raised parts 19 that facilitate positioning and holding of the cannula inside the container.

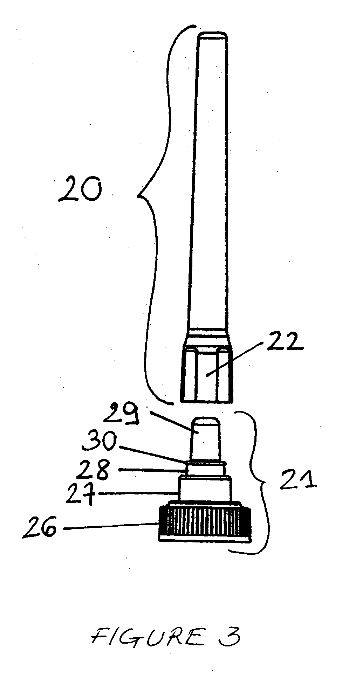

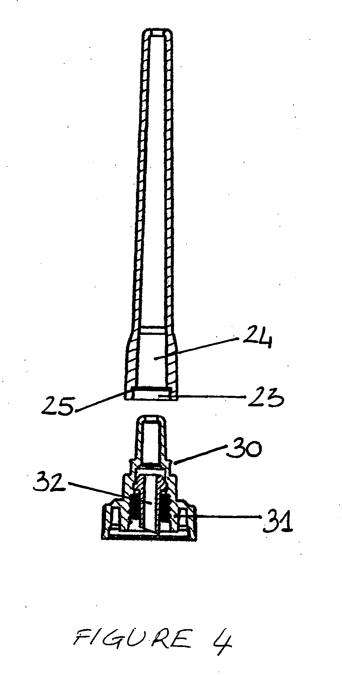

In a particular embodiment, the invention also relates to a cannula, for example for disposable use, constituted by two parts: a long cannula 20 and a short cannula 21 as shown in Figures 3 and 4.

As can be seen in the figures, the long cannula 20 has an essentially truncated-conical or truncated-pyramidal end, 22, the inside of which has a first cylindrical concavity 23 at the edge and a subsequent second concavity, essentially truncated-conical in shape, 24 with a smaller radius than the first concavity.

At the end facing the concavity 24, the concavity 23 has a slight indentation 25.

The short cannula 21 has an outer rim 26 and a central collar 27 extending in a cylindrical extension 28 (with a smaller radius than the radius of the collar) and in a nozzle 29 with an even smaller radius and essentially truncated-conical in shape.

The extension 28 terminates with a projecting circular edge 30 with a radius slightly larger than the radius of the cylindrical concavity 23.

The two cannulae may engage with each other by inserting the nozzle 29 of the short cannula into the end 22 of the long cannula and pushing, so that the edge 30 engages in the indentation 25, coming into contact with the edge of the concavity 24, to make a seal between the short cannula and the long cannula.

Inside the rim 26 of the short cannula 21, and concentric and integral with it, is a cylindrical surface 31, at the centre of which is a small tube 32 coaxial with the nozzle 29.

The outer surface of the rim 26 may be knurled to make it easier for the user to grip at the time of use.

The container and cannula as described above may be produced in any material suitable for the purpose; plastic materials, such as polyethylene, are particularly indicated due to their light weight, low costs and ease of processing.

The use of the cannula according to the invention is evident in the light of the description and the drawings.

After removing the sterile cannula from its container, the user will fit it to the mouth of the package containing the formulation to be dispensed (which is normally sealed by a easily perforable membrane) holding it by the rim 26.

Pressing the small tube 32 of the short cannula 21 against the membrane of the package, the mouth of the package will enter the space between the cylindrical surface 31 and the small tube 32 with pressure, which will thereby break the sealing membrane of the package.

The user may now dispense the medical formulation in the package either through the full cannula (for example for dispensing into body cavities, such as for anal application in the case of haemorrhoids) or, after detaching the long cannula 20, directly through the nozzle 29 of the short cannula 21 if use of the long cannula 20 is not required (for example in the case of application to the outer epidermis).

## Claims

1. Sterile container constituted by an essentially cylindrical body (10), hollow and open at only one end, and by a cap (11) capable of engaging with perfect seal in said open end of the body (10).

2. Container as claimed in claim 1, wherein the cap (11) has a head (12) and a cylindrical extension (13), with a smaller diameter than the head (12), on the outer surface of which is a raised circular part (14).

3. Container as claimed in claims 1 and 2, wherein on the inner surface of the body (10) is a raised part (15), disposed at a distance from the edge slightly below the distance between the lower surface of the head (12) of the cap (11) and the raised circular part (14).

4. Container as claimed in claims 1 - 3, wherein the hollow cylindrical body (10) is shaped so that the end (16) next to the aperture has the dimensions of the cap (11) then tapering in the shape of a cannula (17); and the final part of the cylindrical extension (13) has a final taper (18).

5. Container as claimed in claims 1 - 4, wherein the inner surface of the parts (16) and of the intermediate and final part of the part (17) may have raised parts (19) that facilitate positioning and holding of the cannula inside the container.

6. Cannula constituted by a long cannula (20) and a short cannula (21) wherein said long cannula (20) has an essentially truncated-conical or truncated-pyramidal end, (22), the inside of which has a first cylindrical concavity (23) at the edge and a subsequent second concavity, essentially truncated-conical in shape, (24) with a smaller radius than the first concavity and wherein at the end facing the concavity (24), said concavity (23) has a slight indentation (25).

7. Cannula as claimed in claim 6, wherein the short cannula (21) has an outer rim (26) and a central collar (27) extending in a cylindrical extension (28) (with a smaller radius than the radius of the collar) and in a nozzle (29) with an even smaller radius and essentially truncated-conical in shape and wherein said cylindrical extension (28) terminates with a projecting circular edge (30).

8. Cannula as claimed in claim 7, wherein the radius of the projecting circular edge (30) is slightly larger than the radius of the first concavity (23) and is able to engage firmly with the indentation (25).

9. Cannula as claimed in claim 8, wherein inside the rim (26) of the short cannula (21), and concentric and integral with it, is a cylindrical surface (31) and at the centre of the rim is a small tube (32) coaxial with the nozzle (29).

10. Cannula as claimed in claims 6 - 9, wherein the outer surface of the rim (26) is knurled.

11. Kit composed of a container as claimed in claims 1 - 5 containing a cannula as claimed in claims 6 - 10.

12. Container, cannula and/or kit as claimed in one of the previous claims in plastic material.
